(19) Europäisches Patentamt
European Patent Office
Office européen des brevets

(11) **EP 3 784 047 B1**

(12) **FASCICULE DE BREVET EUROPEEN**

(45) Date de publication et mention de la délivrance du brevet:
**24.07.2024 Bulletin 2024/30**

(21) Numéro de dépôt: **19719320.4**

(22) Date de dépôt: **26.04.2019**

(51) Classification Internationale des Brevets (IPC):
**A23J 1/18** *(2006.01)* **C12N 1/06** *(2006.01)*

(52) Classification Coopérative des Brevets (CPC):
**A23J 1/18; C12N 1/063; C12N 1/066**

(86) Numéro de dépôt international:
**PCT/EP2019/060750**

(87) Numéro de publication internationale:
**WO 2019/207111 (31.10.2019 Gazette 2019/44)**

(54) **PROTEINES DE LEVURES**

HEFEPROTEINE

YEAST PROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **27.04.2018 FR 1853748**

(43) Date de publication de la demande:
**03.03.2021 Bulletin 2021/09**

(73) Titulaire: **Lesaffre et Compagnie**
**75001 Paris (FR)**

(72) Inventeurs:
- **MENIN, Rudy**
  **94600 CHOISY LE ROI (FR)**
- **SPOLAORE, Pauline**
  **94370 SUCY-EN-BRIE (FR)**
- **MOULY, Isabelle**
  **59700 MARCQ-EN-BAROEUL (FR)**

(74) Mandataire: **Bandpay & Greuter**
**11 rue Christophe Colomb**
**75008 Paris (FR)**

(56) Documents cités:
| | |
|---|---|
| **DE-C- 850 286** | **FR-A1- 2 207 985** |
| **GB-A- 554 010** | **US-A- 4 080 260** |
| **US-A1- 2009 123 990** | |

EP 3 784 047 B1

**Description**

La présente invention se rapporte au domaine des protéines issues de microorganismes et plus précisément issues de levures, utilisables de façon large pour la nutrition, le bien-être et la santé des humains et des animaux.

BIBLIOGRAPHIE

Les protéines représentent le composant principal des tissus humains et animaux. Du point de vue nutritionnel, les protéines sont hydrolysées par les protéases et les peptidases en peptides et en acides aminés. Les acides aminés apportent des éléments essentiels pour l'organisme tels que de l'azote, des hydrates de carbone et du soufre. L'organisme ne fixe pas l'azote minéral pour l'incorporer dans des molécules organiques ; les acides aminés apportent donc de l'azote. Les acides aminés soufrés apportent le soufre important pour le métabolisme. Les acides aminés sont également les éléments essentiels pour la synthèse des protéines, des peptides et de substances de faible poids moléculaires physiologiquement indispensables comme le glutathion, la créatine, la dopamine, la sérotonine etc. Aussi les besoins en protéines pour la nutrition humaine et animale grandissent-ils avec l'accroissement de la population mondiale. En conséquence, il est nécessaire de trouver des sources de protéines supplémentaires et/ou alternatives aux protéines animales. Les protéines issues de végétaux (céréales, légumineuses) ou d'insectes sont déjà valorisées. L'obtention de protéines d'origine microbienne fait appel aux mécanismes de fermentation connus depuis des siècles. Les protéines microbiennes sont alors soit des composants de la cellule entière ou de la paroi, soit des protéines isolées. L'un des inconvénients des protéines d'origine microbienne pour l'alimentation humaine est le taux d'acides nucléiques dans les isolats. Ce taux doit en effet être faible parce que l'un des produits finaux du métabolisme des acides nucléiques est l'acide urique que l'organisme humain, dépourvu d'uricase, l'enzyme nécessaire à son catabolisme, ne dégrade pas. D'autres inconvénients sont par exemple le rendement d'extraction et ou la pureté des protéines obtenues par isolement à partir de microorganismes.

Après une lyse cellulaire, par exemple des cellules de levures, les protéines sont classiquement récupérées dans la phase « noble », c'est-à-dire la phase soluble, qui correspond aux extraits de levures et contient les composés riches en goût. Or le goût peut représenter un inconvénient, en fonction de l'application prévue de l'extrait protéique obtenu. Trois brevets d'une même équipe traitent de l'obtention d'extraits concentrés en protéines. Le brevet US3867555 divulgue un procédé permettant d'obtenir des extraits protéiques présentant un faible taux d'acides nucléiques. Les cellules sont rompues mécaniquement (haute pression, ultra-sons) ou chimiquement (autolyse, enzymes externes) de façon à ce que les protéines restent dans la fraction soluble. Ces protéines sont cependant mélangées au résultat de l'hydrolyse, c'est-à-dire à des acides aminés, des peptides et des polysaccharides ou des sucres simples. Après centrifugation pour séparer et écarter la fraction insoluble, les protéines sont précipitées et un traitement alcalin permet l'hydrolyse des acides nucléiques qui sont ensuite éliminés par filtration. Le rendement global de récupération des protéines « non hydrolysées » est cependant faible.

Le brevet US3887431 divulgue l'utilisation de la nucléase endogène présente dans la fraction soluble après lyse des levures pour dégrader les acides nucléiques. Une étape de concentration sous vide permet d'obtenir à la fois un extrait protéique concentré et un produit pauvre en goût et en odeur.

Le brevet US3991215 divulgue un procédé qui, après rupture des cellules, applique sur la fraction cytoplasmique soluble un traitement à ultra haute température qui permet d'obtenir un produit enrichi en protéines, avec peu d'acides nucléiques.

Finalement, il existe plusieurs directions d'amélioration des procédés d'isolement de protéines de microorganismes, dans le but d'obtenir une meilleure pureté, de meilleurs rendements et/ou des extraits protéiques directement utilisables en nutrition ou comme compléments alimentaires. Les documents FR 2 207 985 A1 et US 4 080 260 A décrivent des procédés d'obtention d'extraits de protéines d'une fraction soluble.

RESUME DE L'INVENTION

A contre-courant des techniques communes, les inventeurs ont développé une méthode d'isolement de protéines de microorganismes à partir de la fraction insoluble obtenue après lyse desdits microorganismes. Cette nouvelle méthode permet d'obtenir avec un bon rendement des extraits concentrés de protéines non hydrolysées, pauvres en acides nucléiques, en odeur et en goût.

Le procédé selon l'invention débute par une lyse des cellules préférentiellement par plasmolyse thermique qui a pour conséquences une inactivation des enzymes des microorganismes et la libération dans le milieu des acides aminés libres, dont l'acide glutamique. L'ensemble est alors soumis à une enzyme de type glucanase et une enzyme de type ribonucléase puis soumis à une séparation à l'issue de laquelle la fraction insoluble représente le produit d'intérêt dont le taux de protéines vraies est d'au moins 72%. Selon un autre mode de réalisation de l'invention, une première séparation est effectuée après la phase de plasmolyse thermique et seule la fraction insoluble qui comprend les protéines, les polysaccharides et l'ARN est soumise à l'action d'une glucanase et d'une ribonucléase puis séparée de nouveau pour conserver la fraction insoluble riche en protéines.

L'extrait protéique obtenu est dépourvu d'odeur et de goût, pauvre en acides nucléiques. L'extrait protéique qui contient encore des lipides issus de la membrane peut être délipidé par des méthodes connues de l'homme de l'art telles que l'extraction avec un solvant de type hexane ou éthanol, avec du $CO_2$ supercritique ou un traitement par des lipases ou phospholipases suivi d'une séparation de la phase solubilisée.

Les protéines ainsi obtenues sont directement utilisables pour des applications en nutrition, en compléments alimentaires etc.

DEFINITIONS

Par microorganisme, on entend un organisme vivant invisible à l'œil nu et visible au microscope. Dans le cadre de la présente invention les microorganismes sont préférentiellement des bactéries (microorganismes procaryotes) ou des levures (microorganismes eucaryotes). Levure, au singulier ou au pluriel, est un terme générique désignant des microorganismes eucaryotes aptes à provoquer une fermentation de matières organiques. Parmi les levures, on citera de façon non exhaustive les genres *Saccharomyces, Candida, Pichia, Kluyveromyces.*

Par crème de levure, on désigne la suspension de levures obtenue après multiplication en cuve, et après centrifugation qui permet de séparer ladite suspension du liquide ambiant aussi appelé moût. La multiplication peut aussi être appelée culture ou, par extension, fermentation. Les protéines sont des macromolécules constituées d'un enchaînement d'acides aminés reliés par des liaisons peptidiques. Elles comptent parmi les principaux constituants de base de toutes les cellules animales et végétales. Elles représentent jusqu'à 50% du poids sec d'un être vivant et constituent 15 à 20% de notre apport énergétique quotidien. Les protéines alimentaires et corporelles sont les principales sources d'azote et d'acides aminés, qui ont plusieurs fonctions métaboliques majeures : ce sont des substrats de la synthèse protéique, des précurseurs de composés azotés importants dans l'organisme (acides nucléiques, monoxyde d'azote, glutathion...) et des substrats du métabolisme énergétique.

D'une manière générale, en agro-alimentaire, la teneur en protéines est assimilée au taux d'azote multiplié par 6,25 (le coefficient 6,25 provient de la teneur moyenne en azote d'une protéine qui est de 100/6,25 soit 16%). Par protéines vraies, on entend un taux de protéines plus proche de la réalité, corrigé du biais induit par l'azote non protéique, par exemple l'azote des acides nucléiques. Ainsi, sous forme de formule, le taux de protéines vraies peut être assimilé à azote total diminué de l'azote des acides nucléiques et de l'azote ammoniacal, multiplié par 6,25. Alternativement, le taux de protéines vraies peut être évalué par le dosage des acides aminés totaux. On entend par protéines natives intègres les protéines du microorganisme qui sont dans l'état dans lequel elles se trouvent dans le microorganisme vivant. Par extension, les protéines natives dénaturées ont une conformation spatiale potentiellement modifiée, par repliement ou coagulation. Les protéines peuvent aussi être partiellement ou totalement hydrolysées.

Par plasmolyse, on entend une rupture de l'étanchéité du microorganisme, préférentiellement la levure, ou de son aspect compartimenté. Il y a perte d'eau suite à une perméabilisation de la membrane.

DESCRIPTION DETAILLEE DE L'INVENTION ET DES MODES DE REALISATION

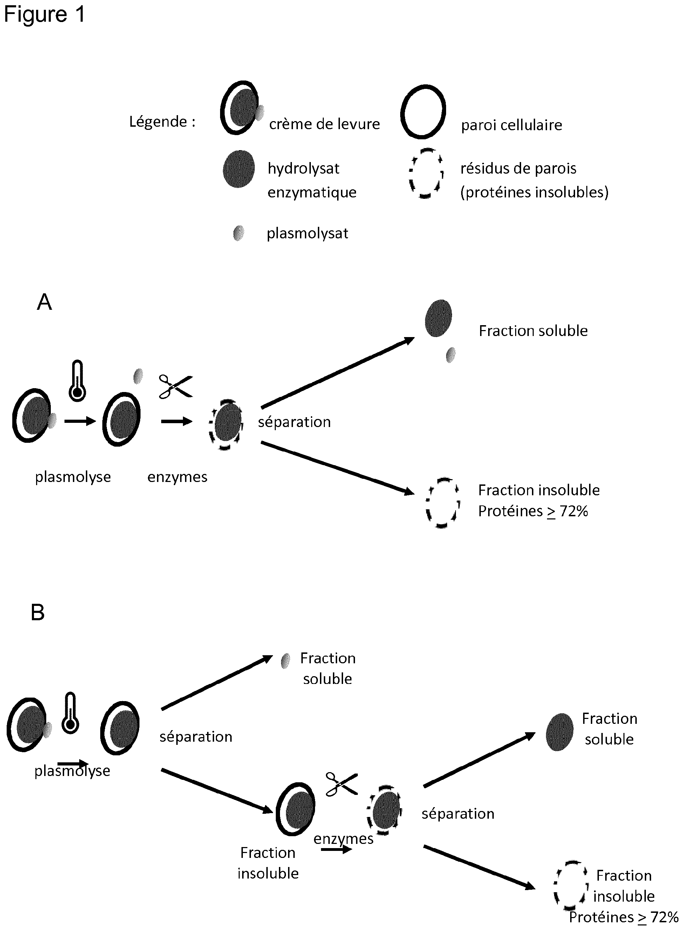

La Figure 1 représente les deux modes de réalisation principaux du procédé selon l'invention.
La Figure 1A illustre le procédé dans lequel il n'y a pas d'étape de séparation entre la plasmolyse et l'hydrolyse enzymatique. La Figure 1B illustre le procédé dans lequel une séparation est effectuée après l'étape de plasmolyse et dans lequel l'hydrolyse enzymatique est alors effectuée sur la fraction insoluble issue de cette séparation.
La Figure 2 présente les profils protéiques respectifs d'un extrait protéique issu d'un procédé selon l'invention, et d'un extrait protéique dit classique issu d'un procédé connu d'extraction de protéines à partir de la fraction soluble obtenue après l'étape de plasmolyse.

Le procédé selon l'invention s'applique à tout type de levures, plus spécifiquement toute levure présentant un historique de consommation en nutrition humaine ou animale. D'un point de vue industriel, le procédé selon l'invention est mis en oeuvre sur une crème de levure telle que définie ci-avant, c'est-à-dire sur une suspension de levures. Préférentiellement les levures sont choisies parmi les levures du genre *Saccharomyces, Pichia, Candida, Kluyveromyces, Yarrowia* ou *Wickerhamomyces.* De façon préférentielle, les levures sont choisies parmi *Saccharomyces cerevisiae, Pichia jadinii (également appelée Candida utilis), Pichia pastoris, Kluyveromyces lactis, Kluyveromyces marxianus, Yarrowia lipolytica, Wickerhamomyces anomalus etc.* Ces levures comprennent entre 6 et 11% d'azote. L'azote est mesuré par la méthode de Kjeldahl connue de l'homme du métier. Comme indiqué plus haut, le taux de protéines vraies est extrapolé à partir de ce dosage d'azote en utilisant le coefficient multiplicateur 6,25.

La culture des levures est mise en oeuvre selon des méthodes connues de l'homme du métier. Un protocole est décrit dans le livre de référence « Yeast technology » par Gerald Reed et Tilak W. Nagodawithana (ISBN 0-442-31892-8) aux pages 284 à 293. Ladite culture, aussi appelée biomasse, est récoltée par centrifugation ou filtration et peut être lavée. On obtient une crème de levure.

Les cellules sont alors soumises à une rupture mécanique ou chimique selon des méthodes connues telles que homogénéisation à haute pression, broyage mécanique, désintégration aux ultrasons, cycles répétés de congélation-décongélation, choc osmotique ou lyse enzymatique. Selon un mode préféré de réalisation de l'invention, les levures sont soumises à une plasmolyse thermique, à une température comprise entre 70 et 95°C adaptée selon le type de levure. La température est préférentiellement comprise entre 80 et 90°C. L'homme du métier saura ajuster la température et/ou la durée de la lyse, par exemple en fonction de la résistance des cellules et/ou des enzymes qu'il souhaite inactiver durant cette étape. La durée sera comprise entre 30 secondes et 3 heures, jusqu'à 4 heures, préférentiellement 1 min., 5 min., 10 min., 30 min., 45 min., 1h, 1 h30, ou 2h, jusqu'à 3, voire 4h. Cette étape de plasmolyse permet une dénaturation de la levure, une inactivation des enzymes endogènes et une perméabilisation de la membrane qui permet de libérer une fraction soluble. La fraction soluble comprend essentiellement les acides aminés libres, des petits peptides, et des minéraux et parmi ces acides aminés, certains qui ont un impact sur le goût tel l'acide glutamique.

Dans un premier mode de réalisation de l'invention, l'ensemble issu de l'étape de plasmolyse et contenant la fraction soluble et la fraction insoluble est alors soumis à une ou plusieurs activités enzymatiques. Cette étape a pour objectif de solubiliser le maximum de constituants qui ne sont pas des protéines, sans attaquer les protéines. De façon préférentielle, on utilisera une activité ribonucléase (EC 3.1.4.1) et une activité glucanase (EC 3.2.1). Les activités enzymatiques peuvent être mises en oeuvre séquentiellement ou simultanément. L'activité ribonucléase va transformer l'ARN en 5' nucléotides et le solubiliser, le faisant ainsi passer dans la fraction soluble. Il est possible d'utiliser plusieurs ribonucléases, et par exemple un mélange d'endo- et d'exo-nucléases. De façon optionnelle, on peut utiliser une déaminase pour transformer l'AMP en IMP, dans le but de valoriser le pouvoir exhausteur de goût de la fraction soluble. L'action d'une ou plusieurs glucanases va permettre de solubiliser les polysaccharides de parois en oligosaccharides solubles. Le temps et la température seront ajustés en fonction des spécifications des enzymes utilisées. La température sera comprise entre 40 et 65°C, de façon préférentielle 60°C. La durée sera comprise entre 8 et 24 heures, préférentiellement entre 16 et 24h, plus préférentiellement 18h. Cette étape enzymatique permet d'obtenir d'une part une nouvelle fraction soluble qui comprend des nucléotides, des polysaccharides (environ 60% de glucides totaux) et une faible proportion d'acides aminés, et d'autre part une fraction insoluble qui comprend essentiellement les protéines.

La dernière étape est une séparation de la fraction soluble et de la fraction insoluble.

Dans un second mode de réalisation de l'invention, les fractions solubles et insolubles issues de l'étape de plasmolyse sont séparées. La fraction soluble sera mise de côté, éliminée et pourra être valorisée comme extrait de levure. La fraction insoluble comprend les écorces (ou parois), des polymères, des polysaccharides, de l'ARN et les protéines coagulées par la chaleur. Cette fraction insoluble est conservée. Elle est alors soumise aux activités enzymatiques ribonucléase et glucanase telles qu'indiquées ci-avant, selon un protocole similaire d'incubation (température, temps) et de séparation des fractions soluble et insoluble. La fraction soluble finale peut être mise de côté et valorisée.

La fraction insoluble finale, issue de la digestion enzymatique, présente un taux de protéines « vraies » supérieur ou égal à 70%, préférentiellement supérieur ou égal à 72%, à 80% et jusqu'à 85%. D'une manière générale, la levure comprend entre 50 et 55% de protéines « vraies ». Le procédé selon l'invention permet d'éliminer de nombreux éléments et d'obtenir un produit plus concentré en protéines. De façon optionnelle, la quantité de lipides dans cette fraction insoluble peut être réduite, soit par l'action de solvants (éthanol, hexane), soit par $CO_2$ supercritique, soit par l'action d'une lipase ou d'une phospholipase. Des puretés protéiques vraies de plus de 80% peuvent alors être obtenues.

Selon un mode de réalisation de l'invention, les actions respectives des ribonucléases et glucanases sont mises en oeuvre de façon séquentielle, sans importance de l'ordre. Selon un mode préféré de réalisation de l'invention, les activités ribonucléase et glucanase sont appliquées en même temps.

Le produit ainsi obtenu peut être lyophilisé, séché par une méthode connue de l'homme du métier, par exemple par atomisation ou sous vide, et conservé en gardant ses propriétés et sa qualité.

Ainsi, selon un mode de réalisation, l'invention porte sur un procédé d'obtention de protéines de levures comprenant les étapes suivantes :

    a) disposer d'une crème de levure ;
    b) exposer cette crème de levure à une plasmolyse thermique à une température comprise entre 70 et 95°C, pendant une durée comprise entre 1 minute et 3 heures, préférentiellement entre 40 minutes et 2 heures ;
    c) soumettre l'ensemble à l'activité d'au moins une ribonucléase et une glucanase, séquentiellement ou simultanément, à une température comprise entre 40 et 65°C pendant une durée comprise entre 8 et 24 heures ;
    d) séparer la fraction insoluble et la fraction soluble,

dans lequel la fraction insoluble recueillie à l'étape d) est dépourvue de goût, présente un taux de nucléotides inférieur à 3% et un taux de protéines vraies d'au moins 72%.

Selon un autre mode de réalisation, l'invention porte sur un procédé d'obtention de protéines de levures comprenant les étapes suivantes :

a) disposer d'une crème de levure ;
b) exposer cette crème de levure à une plasmolyse thermique à une température comprise entre 70 et 95°C, pendant une durée comprise entre 1 minute et 3 heures, préférentiellement entre 40 minutes et deux heures ;
b') séparer la fraction insoluble et la fraction soluble ;
c) soumettre la fraction insoluble à l'activité d'au moins une ribonucléase et une glucanase, séquentiellement ou simultanément, à une température comprise entre 40 et 65°C pendant une durée comprise entre 8 et 24 heures ;
d) séparer la fraction insoluble et la fraction soluble,

dans lequel la fraction insoluble recueillie à l'étape d) est dépourvue de goût, présente un taux de nucléotides inférieur à 3% et un taux de protéines vraies d'au moins 72%.

Dans la suite de la présente description, la fraction insoluble recueillie à l'étape d) pourra aussi être appelée « extrait protéique (de levure) selon l'invention ». *A contrario*, un extrait protéique de levure obtenu, selon un procédé connu de l'art antérieur, à partir de la fraction soluble issue de la plasmolyse, pourra être appelé « extrait protéique classique ».

Selon un mode de réalisation préféré de l'invention, la crème de levure de l'étape a) est issue de la fermentation de levures choisies parmi *Saccharomyces cerevisiae, Pichia,* préférentiellement *Pichia jadinii, Kluyveromyces,* préférentiellement *Kluyveromyces marxianus* ou *Kluyveromyces lactis, Yarrowia,* préférentiellement *Yarrowia lipolytica,* ou *Wickerhamomyces,* préférentiellement *Wickerhamomyces anomalus.* Avantageusement, la levure est choisie parmi *Saccharomyces cerevisiae, Pichia jadinii* ou *Kluyveromyces marxianus.* La levure préférée est *Saccharomyces cerevisiae.*

Selon un mode de réalisation préféré de l'invention, l'étape a) de plasmolyse thermique est réalisée à une température comprise entre 80 et 90°C.

Selon un mode de réalisation préféré de l'invention, les activités glucanase et ribonucléase sont appliquées simultanément.

De façon optionnelle, une activité déaminase peut également être appliquée.

Avantageusement, la crème de levure investie à l'étape a) contient une levure enrichie en sélénium. Une culture de levure enrichie en sélénium peut être effectuée selon une méthode connue de l'homme du métier, par exemple celle décrite dans la demande EP 1478732. Le taux de sélénium dans la culture de levure peut alors atteindre 3 000 ppm voire plus.

Le produit obtenu dans la fraction insoluble présente une teneur en azote exclusivement protéique supérieure ou égale à 11,5% sur matière sèche, c'est-à-dire 72% de protéines vraies (en utilisant le coefficient de conversion de 6,25 indiqué ci-avant). Normalement, la levure a une teneur en azote maximale de 11% en azote, ce qui donne 69% de protéines potentielles. La levure contient également du matériel azoté non protéique (des acides nucléiques ARN/ADN). En soustrayant l'azote nucléique de l'azote total, une levure ne contient plus que 50 à 55% de protéines. Ainsi, le taux minimal de 72% de protéines obtenues dans la fraction insoluble finale est caractéristique du procédé selon l'invention. Dans cette même fraction insoluble, le taux de nucléotides totaux est compris entre 0,8 et 1,5%, jusqu'à 3%, le taux de glucides totaux est de 1 à 8% (dosage à l'anthrone selon une méthode connue de l'homme du métier), les taux respectifs de glucanes sont de 0,2 à 4%, de mannanes de 0,2 à 4%, de lipides de 7 à 15%, de matières minérales 1 à 7%.

Il peut être intéressant de distinguer un extrait protéique de levure obtenu par le procédé selon l'invention d'un extrait selon l'art antérieur, à savoir un extrait protéique obtenu à partir de la fraction soluble après lyse des cellules et ayant subi une étape de concentration, qui présenterait alors un taux de protéines d'au moins 72% et possiblement un taux de nucléotides inférieur à 3%.

Dans ce but, les lipides totaux ont été dosés, sur des extraits protéiques issus de *Saccharomyces cerevisiae,* par une méthode gravimétrique après hydrolyse acide et extraction à l'hexane à l'aide d'un appareil de Soxhlet. Les taux de lipides sont systématiquement supérieurs à 7%. Ainsi, la fraction insoluble recueillie à l'étape d) d'un procédé d'extraction selon l'invention présente en outre un taux de lipides supérieur à 7%. En comparaison, le taux de lipides est inférieur à 1% sur un extrait protéique « classique » de levure. Le taux de lipides peut être différent selon l'espèce ou la souche de levure. D'une manière générale, pour un produit industriel, le microorganisme de départ est indiqué dans la fiche technique. L'homme du métier saura donc extrapoler et déduire une combinaison espèce/taux de lipides dans l'extrait protéique.

Dans le cas d'un extrait protéique de levure selon l'invention qui aurait été soumis à une étape de délipidation, le taux de lipides pourrait ne pas être suffisant pour distinguer les deux origines. Une évaluation du profil de solubilité protéique de l'extrait protéique peut alors compléter l'analyse comparative. Le pourcentage de solubilité est déterminé selon l'équation suivante :

$$\% \ solubilit\acute{e} = \frac{\%\text{N total dans le surnageant}}{\%\text{N initial dans le milieu réactionnel}}$$

où %N désigne le pourcentage d'azote déterminé par la méthode de Kjeldahl.

La solubilité d'un extrait protéique selon l'invention est inférieure à 3,5% dans l'eau.

En comparaison, un extrait protéique « classique » de levure est considéré comme soluble et comprend seulement entre 5 et 10% d'éléments insolubles.

Dans ce même but, les profils de poids moléculaires d'un extrait protéique de levures selon l'invention et d'un extrait protéique selon l'art antérieur ont été comparés. Un extrait protéique de levures obtenu selon l'invention ne présente pas de pic de faible poids moléculaire. L'essentiel du profil protéique est réparti autour de 40-45 kDa. Les composés les plus petits sont à environ 500 Da, ce qui correspond à des petits peptides. En comparaison, le profil d'un extrait protéique « classique » de levure présente plusieurs pics de poids moléculaires et une quantité non négligeable au niveau des faibles poids moléculaires. Il est alors possible d'extrapoler de ces profils et de mesurer le rapport

$$\frac{\text{acides aminés totaux} - \text{acides aminés libres}}{\text{acides aminés totaux}}$$

(acides aminés dosés selon une méthode classique de dosage des acides aminés dans les aliments par exemple la méthode officielle issue du Règlement CE EU152/2009). Ce rapport est proche de 1, toujours supérieur à 0,9 pour un extrait protéique de levure selon l'invention alors qu'il peut être compris entre 0,30 et 0,85 sur des extraits protéiques de levure « classiques ».

Le produit obtenu par le procédé selon l'invention se distingue des extraits protéiques connus par son origine microbienne, autrement dit non végétale et non animale, sa haute teneur en protéines, sa faible teneur en acides nucléiques, les lipides de la membrane cellulaire encore présents si aucun traitement délipidant n'est appliqué. Il est en outre exempt de goût. Son origine microbienne a aussi pour avantage qu'il est issu d'une matière première qui n'est pas connue comme allergène.

Les protéines de levure présentent comme avantage majeur leur qualité nutritionnelle. Ainsi, le produit obtenu par le procédé selon l'invention est une source de protéines qui présente un avantage majeur : sa qualité, représentée par le score PDCAAS (Protein Digestibility Corrected AminoAcids Score) qui est égal à 1, c'est-à-dire similaire à celle des protéines animales de référence telles la caséine et l'ovalbumine. L'évaluation de la qualité des protéines permet de déterminer leur capacité à satisfaire les besoins métaboliques. En conséquence, toute application liée à la nutrition et aux compléments alimentaires ou compléments sportifs peut utiliser un extrait protéique obtenu par le procédé selon l'invention. Les applications citées ici n'ont pas la prétention d'être exhaustives.

Dans le cadre de la nutrition, de la santé et du bien-être humain, un tel extrait protéique peut être utilisé pour le contrôle du poids, comme complément pour les sportifs ou pour les personnes âgées, sous forme de complément alimentaire ou de barre ou de boisson hyper-protéinée. A titre d'exemple, un tel extrait protéique peut être utilisé comme apport de protéines non animales pour des régimes de type vegan, par exemple dans des milk-shakes, des burgers, des nuggets, de la charcuterie végétale, de la farce de raviolis, des boulettes de viande, des flocons d'avoine, des préparations pour assaisonner des pâtes. De même, des pains ou produits de panification riches en protéines peuvent utiliser les extraits protéiques obtenus par le procédé selon l'invention. Comme indiqué précédemment, ces extraits protéiques ont pour avantage de ne pas apporter de goût, d'amertume, ou de notes désagréables (off-notes), caractéristiques de certaines protéines végétales ou d'algues aujourd'hui. En santé humaine, un tel extrait protéique peut être utilisé en nutrition infantile ou en nutrition clinique, c'est-à-dire en nutrition orale ou entérale pour pallier un déséquilibre nutritionnel. Avantageusement, lorsque l'extrait protéique est obtenu à partir d'une crème de levure enrichie en sélénium, il pourra être utilisé comme complément alimentaire stimulant l'immunité et/ou renforçant la qualité de la peau, des cheveux et/ou des ongles.

Enfin, un tel extrait protéique peut être utilisé pour l'apport protéique en alimentation animale. Avantageusement, lorsque l'extrait protéique est obtenu à partir d'une crème de levure enrichie en sélénium, l'animal bénéficiera d'un apport combiné de protéines et de sélénium, ledit sélénium étant biodisponible car intégré dans les protéines, sous forme de séléno-méthionine.

Ainsi, l'invention porte également sur l'utilisation d'un extrait protéique de levures présentant un taux de protéines vraies d'au moins 72%, obtenu selon l'un quelconque des modes de réalisation du procédé selon l'invention, en tant que complément alimentaire pour le contrôle du poids, pour les personnes âgées ou pour les sportifs, sur l'utilisation d'un tel extrait protéique en tant qu'apport de protéines non animales dans des boissons, des produits de panification ou de la charcuterie végétale, sur l'utilisation d'un tel extrait protéique en nutrition clinique, orale ou entérale, ou encore sur l'utilisation d'un tel extrait protéique en nutrition animale. Autrement dit, selon un mode de réalisation, l'invention porte sur un procédé de complémentation alimentaire en nutrition humaine, et/ou en nutrition animale comprenant les étapes consistant à :

- obtenir un extrait protéique de levures en mettant en oeuvre l'un quelconque des modes de réalisation du procédé d'obtention de protéines de levures selon l'invention ; et
- administrer ledit extrait protéique respectivement à un individu humain en tant que complément alimentaire pour le contrôle du poids, pour les personnes âgées ou pour les sportifs, et/ou à un animal en tant qu'apport protéique.

Comme cela a été mentionné précédemment, la fraction soluble finale du procédé selon l'invention peut également être mise de côté et valorisée. Par sa richesse en glucides totaux, elle peut être assimilée à un jus sucré. Le taux de glucides totaux est compris entre 45 et 70%. Ces glucides sont sous forme de glucanes (25 à 40%) et mannanes (25 à 35%), ce qui permet d'utiliser cette fraction soluble pour l'immunostimulation, en particulier pour la santé animale. Suivant le type de glucanase utilisée du glucose libre peut également être libéré. Cette fraction soluble est riche en nucléotides totaux, plus précisément en 5'-GMP et 5'-IMP lorsque l'AMP est transformé en IMP par une deaminase. Ainsi, elle peut être utilisée comme exhausteur de goût. Quand l'AMP n'est pas converti en IMP, le produit peut être utilisé pour masquer de l'amertume ou des off-notes, comme cela est divulgué dans la demande de brevet FR1762074. Sa composition en fait aussi un bon substrat de croissance pour divers microorganismes, en particulier des bactéries.

Les exemples ci-dessous peuvent éclairer la présente invention. Ils ne peuvent pas être considérés comme limitant la portée l'invention.

EXEMPLES

Exemple 1 : Préparation d'un extrait protéique concentré (>75%) à partir de *Saccharomyces cerevisiae*

Réaliser une fermentation de *Saccharomyces cerevisiae* dans des conditions permettant d'atteindre une forte teneur en azote de la levure, 10% d'azote sur matière sèche environ. Utiliser la séparation centrifuge sur le moût de cette fermentation afin d'obtenir une crème de levure à 16-18% de matière sèche-levures puis laver la crème.

Au laboratoire, réaliser une plasmolyse thermique de 3 kg de cette crème lavée riche en azote : monter la température de la crème à une température choisie entre 70 et 95°C à l'aide d'un échangeur puis laisser incuber les 3 kg de crème dans un bécher immergé dans un bain-marie. Laisser la crème sous agitation et maintenir la température de la crème dans le bécher pendant 2h. Baisser ensuite la température du milieu réactionnel à 60°C. Transvaser une fraction dans un erlenmeyer de 500 mL. Immerger l'erlenmeyer dans un bain-marie dont la température est régulée à 60°C. Ajouter les doses d'enzymes suivantes : 0,2% (g pour 100g de crème) d'un mix de deux glucanases et 0,1% d'un mix d'endo et d'exo-ribonucléases. Laisser incuber 18h sous agitation. A la fin de cette incubation, centrifuger. Ecarter la fraction soluble qui contient des nucléotides libres, des polysaccharides et une faible proportion d'acides aminés. La fraction insoluble est l'extrait protéique de levure. Sa composition, mesurée après 3 lavages, est la suivante : 13,0% d'azote, 3% de nucléotides totaux et 4,7% de glucides totaux, soit 77% de protéines vraies.

Exemple 2 : Préparation d'un extrait protéique concentré (75%) à partir de *Saccharomyces cerevisiae* selon une variante du procédé 1.

Dans une variante du procédé présenté dans l'exemple 1, procéder à la séparation de la fraction insoluble du surnageant comprenant les acides aminés libres par centrifugation à la fin de la plasmolyse thermique. La fraction insoluble est alors reprise dans un volume d'eau de ville équivalent au volume de surnageant écarté puis centrifugée à nouveau. Cette opération est effectuée 3 fois au total. Récupérer finalement 2 kg de fraction insoluble à 16% de matière sèche, contenant les protéines, les polysaccharides et le matériel nucléique. Transvaser une fraction dans un erlenmeyer de 500 mL. Immerger l'erlenmeyer dans un bain-marie dont la température est régulée à 60°C et y appliquer la suite du procédé telle que décrite dans l'exemple précédent.

La composition de l'extrait protéique obtenu au final, est la suivante : 12,2% d'azote, 1,4% de nucléotides totaux et 2,9% de glucides totaux, soit 75% de protéines vraies.

Exemple 3 : Préparation d'un extrait protéique concentré (>80%) à partir de *Saccharomyces cerevisiae*

Préparer, grâce aux étapes de plasmolyse thermique, séparation centrifuge puis lavage, la fraction insoluble de *Saccharomyces cerevisiae* contenant protéines, polysaccharides et matériel nucléique selon le protocole décrit à l'exemple 2.

Ajuster l'extrait sec de cette fraction à 14% et le pH au pH optimum des enzymes. Incuber sous agitation 200 g de cette fraction dans un erlenmeyer immergé dans un bain-marie à 60°C pendant 18h en présence des doses d'enzymes suivantes : 0,2% d'une préparation liquide purifiée de glucanase et 0,1% d'un mix d'endo et d'exo-ribonucléases. Récupérer la fraction insoluble par centrifugation et laver à 3 reprises. L'extrait protéique ainsi obtenu comprend alors 13,6% d'azote, 2,4% de nucléotides totaux et 4,2% de glucides totaux soit 83% de protéines vraies.

Exemple 4 : Préparation rapide d'un extrait protéique concentré (75%) à partir de *Saccharomyces cerevisiae*

Dans une variante du procédé présenté dans l'exemple 2, la durée d'incubation peut être réduite à 8h. L'extrait protéique obtenu comprend alors 12,5% d'azote, 3% de nucléotides totaux et 4,4% de glucides totaux soit 75% de protéines vraies.

Exemple 5 : Préparation d'un extrait protéique concentré (80%) à partir de *Saccharomyces cerevisae*

Dans une variante du procédé présenté dans l'exemple 2, l'extrait protéique est séché avant d'être extrait par cinq volumes d'éthanol, lavé puis essoré et séché de nouveau sous vide. L'extrait protéique obtenu comprend alors 13,3% d'azote, 2% de nucléotides totaux et 3,1% de glucides totaux, soit 81% de protéines vraies.

Exemple 6 : Préparation d'un extrait protéique concentré à partir de *Pichia jadinii*

Réaliser une fermentation de *Pichia jadinii* dans des conditions permettant d'atteindre une teneur en azote de la levure de l'ordre de 9% sur matière sèche environ. Utiliser la séparation centrifuge sur le moût de cette fermentation afin d'obtenir une crème de levure à 11-13% de matière sèche puis laver la crème.
Au laboratoire, réaliser une plasmolyse thermique de 3 kg de cette crème lavée riche en azote à une température comprise entre 70 et 95°C, pendant 2h selon le même protocole que celui appliqué à la souche *Saccharomyces cerevisiae*. Récupérer la fraction insoluble par centrifugation puis la laver 3 fois.
Ajuster l'extrait sec de cette fraction à 11-13% et le pH au pH optimum des enzymes. Incuber sous agitation 200 g de cette fraction dans un erlenmeyer immergé dans un bain-marie à 60°C pendant 18h en présence des doses d'enzymes suivantes : 0,2% d'une préparation liquide purifiée de glucanase et 0,1% d'un mix d'endo et d'exo-ribonucléases. Récupérer la fraction insoluble par centrifugation et laver à 3 reprises. L'extrait protéique ainsi obtenu comprend alors 11,7% d'azote, 1,4% de nucléotides totaux et 13,3% de glucides totaux soit 72% de protéines vraies.

Exemple 7 : Préparation d'un extrait protéique concentré à partir de *Kluyveromyces marxianus*.

Réaliser une fermentation de *Kluyveromyces marxianus* dans des conditions permettant d'atteindre une teneur en azote de la levure de l'ordre de 8% sur matière sèche environ. Utiliser la séparation centrifuge sur le moût de cette fermentation afin d'obtenir une crème de levure puis laver la crème. Réaliser une plasmolyse thermique, centrifuger, laver la fraction insoluble et incuber avec un mix d'enzymes contenant des glucanes et des endo et exo-ribonucléases selon le protocole décrit précédemment. Centrifuger une nouvelle fois puis laver la fraction insoluble contenant les protéines.
L'extrait protéique ainsi obtenu comprend 11,8% d'azote, 1,3% de nucléotides totaux et 8,8% de glucides totaux soit 72% de protéines vraies.

Exemple 8 : dosage des lipides totaux dans un extrait protéique « insoluble » obtenu par un procédé selon l'invention et comparaison avec le taux de lipides d'un extrait protéique « classique » de levures, extrait de la partie soluble des levures après lyse cellulaire selon l'art antérieur.

La méthode utilisée est une méthode gravimétrique après hydrolyse acide et extraction à l'hexane à l'aide d'un appareil de Soxhlet. Cette méthode est décrite dans le règlement CE n° 152/2009. Les résultats sont présentés dans le tableau 1 ci-dessous. A partir de trois lots d'extrait protéique obtenu selon l'invention, ces résultats indiquent un taux de lipides voisin de 10 à 11 g pour 100 g d'extrait. En comparaison, un extrait protéique de levures selon l'art antérieur, obtenu à partir de la fraction soluble des levures lysées présentera un taux de lipides inférieur à 1 g pour 100 g d'extrait.

| | Extrait protéique selon l'invention (sur fraction insoluble) | | | Extrait protéique selon l'art antérieur (sur fraction soluble) |
|---|---|---|---|---|
| | lot 1 | lot 2 | lot 3 | |
| Lipides totaux (g/ 100g) | 11,2 | 10,8 | 11,1 | <1 |

Exemple 9 : détermination du profil de solubilité protéique.

Sur un échantillon issu du lot 1 d'extrait protéique selon l'invention (Cf exemple 8), le profil de solubilité protéique a été déterminé à trois valeurs distinctes de pH en solution aqueuse et en utilisant une concentration protéique finale de 2% (m/v). Après 60 minutes de solubilisation sous agitation à température ambiante, le surnageant est récupéré par centrifugation et la teneur en azote total dans le surnageant est déterminée par la méthode de Kjeldahl selon la norme NF EN ISO 5983-2. Pour chaque valeur de pH, le pourcentage de solubilité est déterminé selon l'équation suivante :

$$\%solubilité = \frac{\%N \text{ total dans le surnageant}}{\%N \text{ initial dans le milieu réactionnel}}$$

Le résultat obtenu est inférieur à 3,5%.

Exemple 10 : détermination des profils de poids moléculaire respectivement d'un extrait protéique selon l'invention et d'un extrait protéique « classique ».

Comme dans l'ensemble de ce qui précède, « extrait protéique selon l'invention » désigne un extrait protéique de levure obtenu à partir de la fraction insoluble de cellules de levures lysées. *A contrario*, un « extrait protéique de levure 'classique' » désigne des protéines de levures obtenues par extraction à partir de la fraction soluble de cellules de levures lysées. Extraction selon des méthodes connues de l'homme du métier.

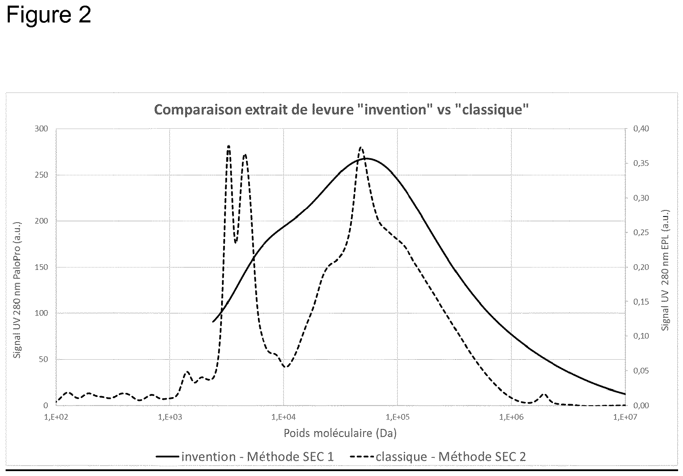

Comme cela est indiqué à l'exemple 9, l'extrait protéique selon l'invention n'est pas soluble en condition aqueuse. Des conditions spécifiques de solubilisation et d'analyse par chromatographie d'exclusion de taille ont été développées. Le protocole est le suivant (méthode SEC1 sur la Figure 2) :

- solubilisation de 10 mg de l'échantillon dans 5 mL de la phase mobile (sous agitation pendant 48h à 90°C),
- analyse de la solution obtenue sur colonne Agilent® PLgel 20 $\mu$m MIXED-A (2 000 à 40 000 000 g/mol, PS équivalent) à 40°C.

Phase mobile : DMSO/LiCl 0,25 M.

Détecteurs RI (refractive index) et UV (280 nm).

La méthode (méthode SEC2 sur la Figure 2) utilisée pour l'extrait protéique de levure « classique » est issue de la monographie de l'OIV (Organisation Internationale du Vin) :

- solubilisation de l'échantillon dans l'eau ;
- analyse sur colonne SUPERDEX 200 10/300 GL (10 000 à 600 000 Da, globular protein équivalent ; 1 000 à 100 000 g/mol, dextran équivalent).

Phase mobile : tampon phosphate + NaCl 0,25 M, pH 7,2

Détection UV à 214, 260 et 280 nm.

Les profils protéiques respectifs sont présentés sur la Figure 2.

Le profil de l'extrait protéique de levure selon l'invention est réparti autour de 45 kDa et ne présente pas de pic de petit poids moléculaire (intervalle 2-10 kDa). En comparaison, le profil d'un extrait protéique de levure « classique » est significativement différent ; il présente deux pics nets dans l'intervalle des faibles poids moléculaires, 2-10 kDa et globalement un profil avec des pics plus nets et plus répartis.

Le dosage des acides aminés totaux et des acides aminés libres selon la méthode officielle issue du Règlement CE EU152/2009 permet également de déterminer le rapport

$$\frac{\text{acides aminés totaux} - \text{acides aminés libres}}{\text{acides aminés totaux}}$$

Celui-ci est très proche de 1 (ou 100%) et toujours supérieur à 0,9 (ou 90%) sur un extrait protéique de levure selon l'invention (détermination sur les 3 lots dont les lipides ont été dosés dans l'exemple 8). Il est classiquement compris entre 0,30 et 0,85 (30 à 85%) sur des extraits protéiques de levures « classiques » du Déposant, et des extraits disponibles dans le commerce.

Exemple 11 : Préparation d'un extrait protéique concentré (>75%) à partir d'une culture de *Saccharomyces cerevisiae* enrichie en sélénium.

Un lot de crème de levure enrichie en sélénium a été soumis au procédé d'extraction de protéines tel que décrit dans l'exemple 1 ou dans l'exemple 2. Le taux de sélénium dans cette crème de levure était proche de 3 200 ppm.
Le taux d'azote total dans l'extrait protéique obtenu était de 13,5%, le taux de protéines vraies de 76,7%. La teneur en sélénium total était de 4 750 ppm (mg Se/kg sec). La teneur en séléno-méthionine était de 84% (Eq Se/Se tot). En comparaison, la levure séléniée SelSaf®3000, source de sélénium organique biodisponible, présente une teneur de 63% en séléno-méthionine.

## Revendications

1. Procédé d'obtention de protéines de levures comprenant les étapes suivantes :

   a) disposer d'une crème de levure ;
   b) exposer cette crème de levure à une plasmolyse thermique à une température comprise entre 70 et 95 °C pendant une durée comprise entre 30 secondes et 4 heures, préférentiellement entre 1 minute et 3 heures, préférentiellement entre 40 minutes et 2 heures ;
   c) soumettre l'ensemble à l'activité d'au moins une ribonucléase et une glucanase, séquentiellement ou simultanément, à une température comprise entre 40 et 65°C, préférentiellement 60°C pendant une durée comprise entre 8 et 24h, préférentiellement 18h ;
   d) séparer la fraction insoluble et la fraction soluble ;

   dans lequel la fraction insoluble recueillie à l'étape d) est dépourvue de goût, présente un taux de nucléotides inférieur à 3% et un taux de protéines vraies d'au moins 72%.

2. Procédé d'obtention de protéines de levures comprenant les étapes suivantes :

   a) disposer d'une crème de levure ;
   b) exposer cette crème de levure à une plasmolyse thermique à une température comprise entre 70 et 95°C pendant une durée comprise entre 30 secondes et 4 heures, préférentiellement entre 1 minute et 3 heures, préférentiellement entre 40 minutes et 2 heures ;
   b') séparer la fraction insoluble et la fraction soluble ;
   c) soumettre la fraction insoluble à l'activité d'au moins une ribonuclease et une glucanase, séquentiellement ou simultanément, à une température comprise entre 40 et 65°C, préférentiellement 60°C pendant une durée comprise entre 8 et 24h, préférentiellement 18h ;
   d) séparer la fraction insoluble et la fraction soluble ;

   dans lequel la fraction insoluble recueillie à l'étape d) est dépourvue de goût, présente un taux de nucléotides inférieur à 3% et un taux de protéines vraies d'au moins 72%.

3. Procédé selon la revendication 1 ou 2 dans lequel une activité déaminase est également appliquée à l'étape c).

4. Procédé selon l'une des revendications 1 à 3 dans lequel les levures sont choisies parmi les espèces *Saccharomyces, Pichia, Candida, Kluyveromyces, Yarrowia, Wickerhamomyces.*

5. Procédé selon la revendication 4 dans lequel les levures sont choisies parmi *Saccharomyces cerevisiae, Pichia jadinii, Kluyveromyces marxianus.*

6. Procédé selon l'une des revendications 1 à 5 dans lequel les enzymes utilisées à l'étape c) sont mises en oeuvre simultanément.

7. Procédé selon l'une des revendications 1 à 6 dans lequel la fraction insoluble recueillie à l'étape d) contient en outre un taux de lipides supérieur à 7%.

8. Procédé selon l'une des revendications 1 à 7 dans lequel la fraction insoluble recueillie à l'étape d) est traitée avec de l'éthanol, un solvant ou du $CO_2$ supercritique pour éliminer les lipides et augmenter le taux de protéines vraies à 80%.

9. Procédé selon l'une des revendications 1 à 8, dans lequel la crème de levure mise en oeuvre à l'étape a) contient une levure enrichie en sélénium.

10. Utilisation d'un extrait protéique de levures présentant un taux de protéines vraies d'au moins 72%, obtenu selon le procédé objet de l'une des revendications 1 à 9, en nutrition humaine.

11. Utilisation selon la revendication 10 en tant que complément alimentaire pour le contrôle du poids, pour les personnes âgées, ou pour les sportifs.

12. Utilisation selon la revendication 10 en tant qu'apport de protéines non animales dans des boissons, des produits de panification ou de la charcuterie végétale.

13. Utilisation selon la revendication 10 en nutrition clinique, orale ou entérale.

14. Utilisation d'un extrait protéique de levures présentant un taux de protéines vraies d'au moins 72%, obtenu selon le procédé objet de l'une des revendications 1 à 9, en nutrition animale.

**Patentansprüche**

1. Verfahren zur Gewinnung von Hefeproteinen, das die folgenden Schritte umfasst:

    a) Bereitstellen einer Hefecreme;
    b) Aussetzen dieser Hefecreme einer thermischen Plasmolyse bei einer Temperatur zwischen 70 und 95 °C während einer Dauer zwischen 30 Sekunden und 4 Stunden, vorzugsweise zwischen 1 Minute und 3 Stunden, vorzugsweise zwischen 40 Minuten und 2 Stunden;
    c) Unterziehen des Ganzen der Aktivität von mindestens einer Ribonuclease und einer Glucanase, sequentiell oder gleichzeitig, bei einer Temperatur zwischen 40 und 65 °C, vorzugsweise 60 °C während einer Dauer zwischen 8 und 24 Stunden, vorzugsweise 18 Stunden;
    d) Trennen der unlöslichen Fraktion und der löslichen Fraktion;

    wobei die in Schritt d) gewonnene unlösliche Fraktion geschmacklos ist, einen Nukleotidgehalt unter 3 % und einen Gehalt echter Proteine von mindestens 72 % aufweist.

2. Verfahren zur Gewinnung von Hefeproteinen, das die folgenden Schritte umfasst:

    a) Bereitstellen einer Hefecreme;
    b) Aussetzen dieser Hefecreme einer thermischen Plasmolyse bei einer Temperatur zwischen 70 und 95 °C während einer Dauer zwischen 30 Sekunden und 4 Stunden, vorzugsweise zwischen 1 Minute und 3 Stunden, vorzugsweise zwischen 40 Minuten und 2 Stunden;
    b') Trennen der unlöslichen Fraktion und der löslichen Fraktion;
    c) Unterziehen der unlöslichen Fraktion der Aktivität von mindestens einer Ribonuclease und einer Glucanase, sequentiell oder gleichzeitig, bei einer Temperatur zwischen 40 und 65 °C, vorzugsweise 60 °C während einer Dauer zwischen 8 und 24 Stunden, vorzugsweise 18 Stunden;
    d) Trennen der unlöslichen Fraktion und der löslichen Fraktion;

    wobei die in Schritt d) gewonnene unlösliche Fraktion geschmacklos ist, einen Nukleotidgehalt unter 3 % und einen Gehalt echter Proteine von mindestens 72 % aufweist.

3. Verfahren nach Anspruch 1 oder 2, wobei eine Deaminaseaktivität ebenfalls in Schritt c) angewendet wird.

**4.** Verfahren nach einem der Ansprüche 1 bis 3, wobei die Hefen aus den Spezies *Saccharomyces, Pichia, Candida, Kluyveromyces, Yarrowia, Wickerhamomyces* ausgewählt sind.

**5.** Verfahren nach Anspruch 4, wobei die Hefen aus *Saccharomyces cerevisiae, Pichia jadinii, Kluyveromyces marxianus* ausgewählt sind.

**6.** Verfahren nach einem der Ansprüche 1 bis 5, wobei die in Schritt c) verwendeten Enzyme gleichzeitig angewendet werden.

**7.** Verfahren nach einem der Ansprüche 1 bis 6, wobei in Schritt d) gewonnene unlösliche Fraktion ferner einen Lipidgehalt über 7 % enthält.

**8.** Verfahren nach einem der Ansprüche 1 bis 7, wobei die in Schritt d) gewonnene unlösliche Fraktion mit Ethanol, einem Lösungsmittel oder superkritischem $CO_2$ behandelt wird, um die Lipide zu entfernen und den Gehalt echter Proteine auf 80 % zu erhöhen.

**9.** Verfahren nach einem der Ansprüche 1 bis 8, wobei die in Schritt a) verwendete Hefecreme eine mit Selen angereicherte Hefe enthält.

**10.** Verwendung eines Hefeproteinextrakts, der einen Gehalt echter Proteine von mindestens 72 % aufweist, erhalten gemäß dem Verfahren nach einem der Ansprüche 1 bis 9, als Nahrungsmittel für Menschen.

**11.** Verwendung nach Anspruch 10 als Nahrungsergänzung zur Gewichtskontrolle, für Senioren oder für Sportler.

**12.** Verwendung nach Anspruch 10 als nicht-tierische Proteinzufuhr in Getränken, Produkten zur Brotherstellung und pflanzlichen Wurstwaren.

**13.** Verwendung nach Anspruch 10 zur klinischen, oralen oder enteralen Ernährung.

**14.** Verwendung eines Hefeproteinextrakts, der einen Gehalt echter Proteine von mindestens 72 % aufweist, erhalten gemäß dem Verfahren nach einem der Ansprüche 1 bis 9, als Tierfutter.

**Claims**

**1.** A method for obtaining yeast proteins, comprising the following steps:

a) providing a yeast cream;
b) exposing this yeast cream to thermal plasmolysis at a temperature of between 70 and 95 °C for a time of between 30 seconds and 4 hours, preferably between 1 minute and 3 hours, preferably between 40 minutes and 2 hours;
c) subjecting the whole to the activity of at least one ribonuclease and one glucanase, sequentially or simultaneously, at a temperature of between 40 and 65 °C, preferably 60°C for a time of between 8 and 24h, preferably 18h;
d) separating the insoluble fraction and soluble fraction;

wherein the insoluble fraction collected at step d) is taste-free, has a nucleotide content of less than 3 % and true protein content of at least 72 %.

**2.** A method for obtaining yeast proteins comprising the following steps:

a) providing a yeast cream;
b) exposing this yeast cream to thermal plasmolysis at a temperature of between 70 and 95 °C for a time of between 30 seconds and 4 hours, preferably between 1 minute and 3 hours, more preferably between 40 minutes and 2 hours;
b') separating the insoluble fraction and soluble fraction;
c) subjecting the insoluble fraction to the activity of at least one ribonuclease and one glucanase, sequentially or simultaneously, at a temperature of between 40 and 65 °C, preferably 60° C for a time of between 8 and 24h, preferably 18h;
d) separating the insoluble fraction and soluble fraction;

wherein the insoluble fraction collected at step d) is taste-free, has a nucleotide content of less 3 % and true protein content of at least 72 %.

**3.** The method according to claim 1 or 2, wherein deaminase activity is also applied at step c).

**4.** The method according to one of claims 1 to 3, wherein the yeasts are selected from among the species *Saccharomyces, Pichia, Candida, Kluyveromyces, Yarrowia, Wickerhamomyces.*

**5.** The method according to claim 4, wherein the yeasts are selected from among *Saccharomyces cerevisiae, Pichia jadinii, Kluyveromyces marxianus.*

**6.** The method according to one of claims 1 to 5, wherein the enzymes used at step c) are used simultaneously.

**7.** The method according to one of claims 1 to 6, wherein the insoluble fraction collected at step d) also has a lipid content higher than 7 %.

**8.** The method according to one of claims 1 to 7, wherein the insoluble fraction collected at step d) is treated with ethanol, a solvent or supercritical $CO_2$ to remove the lipids and increase the true protein content to 80 %.

**9.** The method according to one of claims 1 to 8, wherein the yeast cream used at step a) contains a selenium-enriched yeast.

**10.** Use of a yeast protein extract having a true protein content of at least 72 %, obtained with the method of one of claims 1 to 9, in human nutrition.

**11.** The use according to claim 10 as a food supplement for weight control, for the elderly, or for sportspersons.

**12.** The use according to claim 10 as supply of non-animal protein in beverages, breadmaking products or plant-based deli meats.

**13.** The use according to claim 10 in oral or enteral clinical nutrition.

**14.** Use of a yeast protein extract having a true protein content of at least 72 %, obtained with the method of one of claims 1 to 9, in animal nutrition.

# Figure 1

Légende :   crème de levure   paroi cellulaire

hydrolysat enzymatique   résidus de parois (protéines insolubles)

plasmolysat

A

plasmolyse   enzymes   séparation

Fraction soluble

Fraction insoluble
Protéines $\geq$ 72%

B

plasmolyse   séparation

Fraction soluble

Fraction insoluble   enzymes   séparation

Fraction soluble

Fraction insoluble
Protéines $\geq$ 72%

# Figure 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3867555 A
- US 3887431 A
- US 3991215 A
- FR 2207985 A1
- US 4080260 A
- EP 1478732 A
- FR 1762074

**Littérature non-brevet citée dans la description**

- **GERALD REED ; TILAK W. NAGODAWITHANA.** *Yeast technology,* ISBN 0-442-31892-8, 284-293